Europäisches Patentamt

European Patent Office

Office européen des brevets

(11) Publication number: **0 055 394**
**B1**

# EUROPEAN PATENT SPECIFICATION

(45) Date of publication of patent specification: **13.03.85**

(51) Int. Cl.⁴: **A 61 B 1/12**

(21) Application number: **81109861.5**

(22) Date of filing: **24.11.81**

(54) Endoscope.

(30) Priority: **26.12.80 JP 185944/80**

(43) Date of publication of application:
**07.07.82 Bulletin 82/27**

(45) Publication of the grant of the patent:
**13.03.85 Bulletin 85/11**

(84) Designated Contracting States:
**AT BE CH DE FR GB IT LI NL SE**

(56) References cited:
**EP-A-0 037 191**
**DE-A-1 921 186**
**DE-A-2 920 724**
**US-A-2 244 997**
**US-A-3 903 877**
**US-A-3 949 780**
**US-A-3 958 566**
**US-A-4 198 958**

(73) Proprietor: **OLYMPUS OPTICAL CO., LTD.**
**43-2, 2-chome, Hatagaya Shibuya-ku**
**Tokyo 151 (JP)**

(72) Inventor: **Yabe, Hisao**
**4-22-13, Owada-machi**
**Hachioji-shi Tokyo (JP)**

(74) Representative: **KUHNEN & WACKER**
**Patentanwaltsbüro**
**Schneggstrasse 3-5 Postfach 1729**
**D-8050 Freising (DE)**

Courier Press, Leamington Spa, England.

## Description

This invention relates to an endoscope provided with means for supplying air and water to the control section of the endoscope and means for changing over these operations, as specified in the opening portion of claim 1. Such an endoscope is disclosed in US—A—3903877.

An endoscope generally has an observation window formed at a distal end of an insertion section. Where the insertion section is taken into a coeliac cavity of the human body, a coeliac filth or viscous liquid is likely to be deposited on the observation window, thereby obstructing a field of view. To avoid this difficulty, a fluid passage is provided in the conventional endoscope to clean the observation window by selectively letting water or air be sprayed thereon through said fluid passage. Air or water is also supplied to expand or clean the coeliac cavity.

A valve device for changing over the air supply and water supply operations is formed of a cylinder and piston and set in an endoscope control section. A non-return valve is provided in the air passage of said changeover valve device. The non-return valve prevents air, water, or coeliac viscous in the coeliac cavity from flowing backward.

The material of the non-return valve is deteriorated with time. Or when the coeliac viscous liquid or coeliac filth is solidified, the coeliac filth tends to be tightly attached to the valve seat, thereby preventing the non-return valve from being opened. To avoid the above-mentioned difficulties, there has been proposed a device for forcefully opening the non-return valve by inserting, for example, a rod from the outside of the endoscope into the valve device. However, the material of the non-return valve has such a nature that once the non-return valve is made ready to be tightly attached to the valve seat due to the coagulation of the coeliac fluid, said non-return valve tends to be again tightly fixed to the valve seat even when forcefully taken off therefrom. Therefore, the required repeated removal of the non-return valve gives rise to great inconvenience, and renders said valve unreliable with respect to the function of suppressing the counter flow of the air or water or the unnecessary flow of the coeliac fluid.

Where the soiled non-return valve material is taken out for cleaning, the whole valve device has to be dismembered. Consequently, difficulties are presented in always keeping the material of the non-return valve clean.

It is accordingly an object of this invention to provide an endoscope which unfailingly prevents the material of a non-return valve member included in air- and water-supply operation changeover means from being tightly attached to the valve seat and enables the material of said non-return valve member to be removed together with a piston for washing.

To attain the above-mentioned object, this invention provides an endoscope according to claim 1.

This invention can be more fully understood from the following detailed description when taken in conjunction with the accompanying drawings, in which:

Fig. 1 is a schematic view of an endoscope according to one embodiment of this invention;

Fig. 2 is a sectional view of an air supply and water supply changeover valve device shown in Fig. 1; and

Figs. 3 to 6 are sectional views of an air supply and water supply changeover valve device of an endoscope according to another embodiments of the invention.

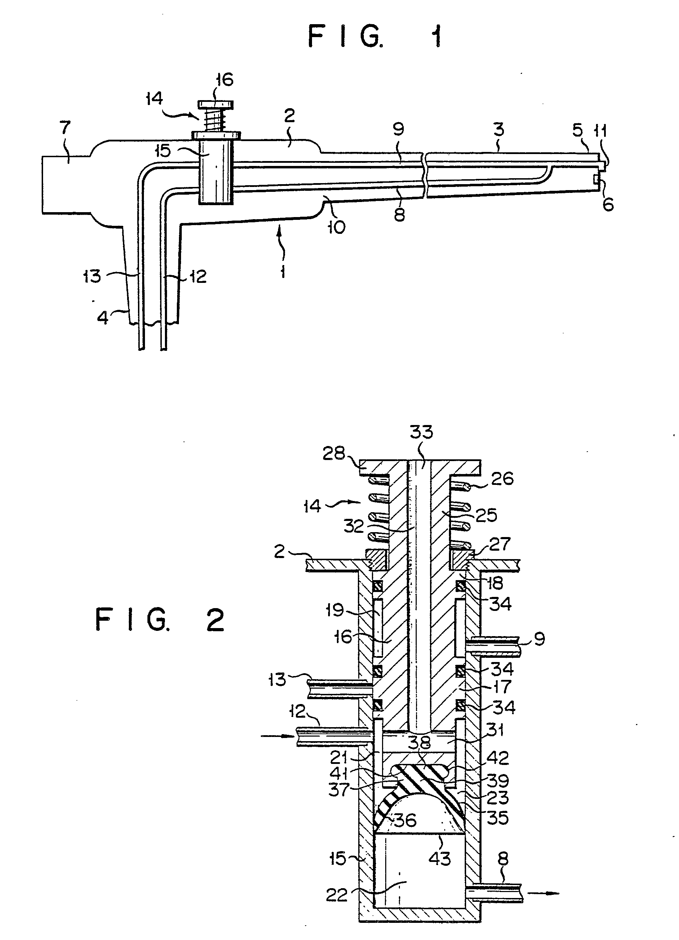

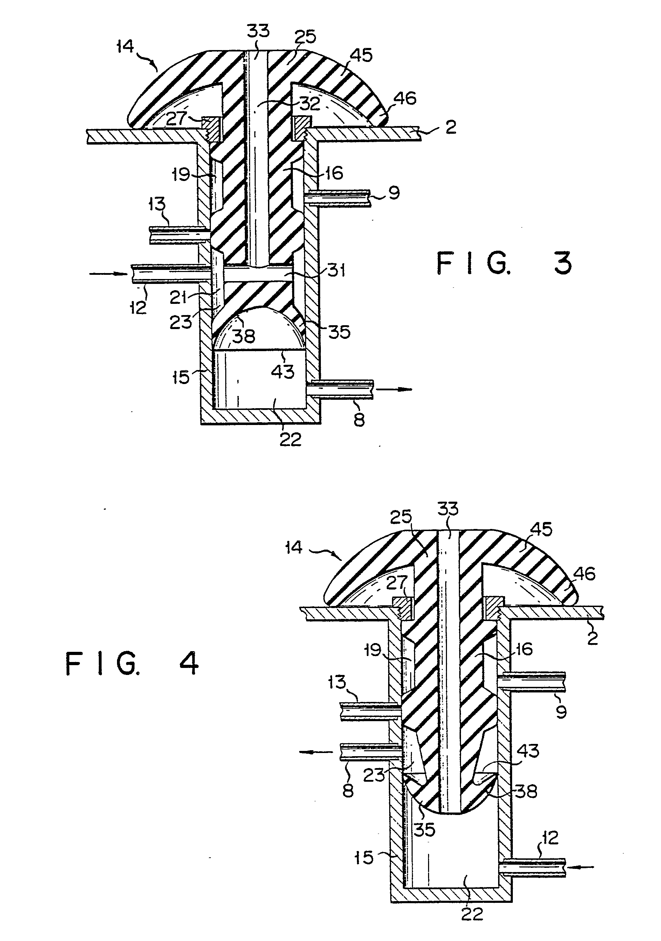

Fig. 1 schematically shows an endoscope 1 according to one embodiment of this invention. As is well known, the endoscope 1 has a control section 2 and an elongate flexible insertion section 3 which is taken into the coeliac cavity of the human body. The proximal end 10 of the insertion section 3 is connected to the control section 2. One end of a universal cord 4 is connected to the control section 3. An image guide and light guide (neither shown) extend through the control section 2 and insertion section 3. One end of the image guide is connected to an observation window 6 set in the distal end 5 of the insertion section 3. The other end of the image guide is connected to an eyepiece 7 of the control section 2. The image guide optically connects the observation window 6 to the eyepiece 7 to observe the coeliac condition at the eyepiece 7.

One end of the light guide is connected to the distal end 5 of the insertion section 3, and the other end of said light guide extends through the universal cord 4 to be connected to a light source unit (not shown).

An air guide tube 8 and water guide tube 9 both acting as fluid-guiding means extend lengthwise through the insertion section 3. The air guide tube 8 and water guide tube 9 are brought together near the distal end 5 of the insertion section 3, and are connected to a nozzle 11 at the distal end 5. The nozzle 11 is open to the surface of the observation window 6. As later described, the nozzle 11 causes air supplied through the air guide tube 8 and water conducted through the water guide tube 9 to be sprayed on the surface of the observation window 6 for cleaning. An air supply tube 12, a water supply tube 13 and a changeover valve device 14 are provided in the control section 2, the air supply tube 12 and water supply tube 13 being extending through the universal cord 4. The air tube 12 conducts into a cylinder 15 of the valve device 14 the compressed air delivered from an air source (not shown) connected to the universal cord 4. The water supply tube 13 allows for the passage into the cylinder 15 of the pressurized water supplied from a water source (not shown) connected to the universal cord 4.

This changeover valve device 14 selectively connects an air supply tube 12 or water supply tube 13 set in the control section 2 to the corresponding air guide tube 8 or water guide tube 9 which are received in the insertion section 3 to act as fluid guide means. The air supply and water supply changeover valve device 14 has concrete arrangement shown in Fig. 2. This changeover valve device 14 comprises the cylinder 15 fitted to the control section 2 and a piston 16 slidably inserted into said cylinder 15. The cylinder 15 is closed at one end and open at the other end to the outside of the endoscope 1. A piston 16 is held in the cylinder 15. The two portions of the peripheral wall of the piston 16 which are axially spaced from each other are chosen to have a larger diameter than the other portions to provide first and second annular partitioning projected portions 17, 18. A water supply conduit 19 is formed in the peripheral wall of that section of the piston 16 which is defined between the paired partitioning projected portions. Said water supply conduit 19 extends in the circumferential direction of the piston 16. An air conduit space 23 is formed by a space 21 defined between the peripheral wall of the piston 16 which is positioned more inward than the first partitioning projected portion 17, the inner wall of the cylinder 15, and the bottom space 22 of the cylinder 15. The head 25 of the piston 16 projects from the cylinder 15. A compression coil spring 26 is wound about the outer peripheral wall of the piston head 25 to urge the piston 16 back to the ready or waiting position. A fixing ring 27 is detachably fitted to the open end of the cylinder 15 by being threadedly inserted, for example, into the inner peripheral wall of said opening of the cylinder 15. The fixing ring 27 abuts against the second partitioning stepped portion 18 of the piston 16 urged by the compression coil spring 26 to cause the piston 16 to be kept waiting in a position shown in Fig. 2. The compression coil spring 26 is interposed between the fixing ring 27 and a flange 28 formed at the outer end of the piston head 25 of the piston 16. When the fixing ring 27 is taken off, the piston 16 can be drawn out of the cylinder 15.

A leak hole 33 is formed in the piston 16 for communication with the outside of the endoscope 1. This leak hole 33 comprises a hole 31 crosswise penetrating the piston 16 to be open to the air conduit space 23 and a hole 32 which communicates with the hole 31, and extends axially of the piston 16 up to the piston head 25 to be open to the outside of the endoscope 1.

The lateral wall of the cylinder 15 is connected to the air guide tube 8 communicating with the bottom space 22 of the cylinder 15 and the water guide tube 9 communicating with the water supply conduit 19 of the piston 16 when kept in a waiting position. The lateral wall of the cylinder 15 is connected to the air supply tube 12 communicating with the air conduit 23 of the piston 16 when kept in a waiting position and the water supply tube 13 which is open to the peripheral wall of the first partitioning projected portion 17 when kept in a waiting position. Where, therefore, the piston 16 is set in a waiting position, then the opening of the water supply tube 13 is closed by the peripheral wall of the first partitioning projected portion 17.

A ring-shaped packing 34 is fitted in recesses of the partition projected portions 17, 18 to assure their airtightness. A non-return valve member 35 is inserted in the cylinder 15 and is fitted to the end of the piston 16. This non-return valve member 35 is prepared from a material having a proper elasticity such as rubber, or synthetic resin in the form of a cup. Said non-return valve member 35 is formed of a flared portion 36, stem portion 37 and base portion 38. The stem portion 37 and base portion 38 jointly constitute an engagement section 39. A hole 41 complementary to said engagement section 39 is formed in the end portion of the piston 16. This hole 41 constitutes an engagement receptacle 42 into which said engagement section 39 is tightly fitted for engagement. The outer edge 43 of the flared portion 36 is set in the inner end of the cylinder 15, and elastically tends to expand, and consequently is tightly pressed against the inner peripheral wall of the cylinder 15. When kept in a waiting position, the non-return valve member 35 is positioned between the opening of the air guide tube 8 and that of the air supply tube 12 in the air conduit space 23 of the cylinder 15, thereby separating said air conduit space 23 into the space 21 and the bottom space.

Where, with the air supply and water supply changeover valve device 14 arranged as described above, the fingers are released from the piston 16, to set said changeover valve device 14 in a waiting position, then the air supply tube 12 is open to the atmosphere through the leak hole 33. Therefore, compressed air delivered from said air supply tube 12 escapes through the leak hole 33, and is not sent to the air guide tube 8 of the insertion section 3. While the air supply and water supply changeover valve device 14 is kept in a waiting position, the opening of the water supply tube 13 is closed by the peripheral wall of the first partitioning projected portion 17, allowing no water supply. Where the piston head 25 of the piston 16 is closed with the finger tip, and the leak hole 33 is closed without pushing the piston 16 thereinto, then the compressed air supplied from the air supply tube 12 forcefully opens the flared portion 36 of the non-return valve member 35 and is delivered to the air guide tube 8 to be ejected from the nozzle 11. Since, at this time, the opening of the water supply tube 13 remains closed, no water is supplied.

When the piston 16 is pushed while the leak hole 33 is closed with the fingers, then the opening of the air supply tube 12 is closed by the peripheral wall of the first partitioning projected portion 17, thereby stopping air supply. Instead, the opening of the water supply tube 13 communicates with that of the water guide tube 9 through the water conduit 19, causing the water duct 13 and water guide tube 9 to communicate with each other. The water supplied from the water source through the water supply tube 13 is conducted through the water guide tube 9 to be sprayed through the nozzle 11, thereby washing the surface of the observation window 6 set at the distal end of the insertion section 3.

The interior pressure of the coeliac cavity is progressively increased, as air or water is taken thereinto. As a result, water, air or a viscous liquid held in the coeliac cavity tends to run backward through the air guide tube 8 of the insertion section 3. At this time, however, the outer edge 43 of the flared portion 36 of the non-return valve member 35 is pressed against the inner wall of the cylinder 15 to prevent said backward flow of air, water or coeliac fluid. Where the fingers are released from the head 25 of the piston 16, then the piston 16 is urged to return the waiting or ready position by the spring force of the coil spring 26.

With the foregoing embodiment, the non-return valve member 35 fitted to the piston 16 frequently slides with the piston 16, thereby preventing the non-return valve member 35 from being tightly attached to the inner wall of the cylinder 15. Even if the non-return valve member 35 should temporarily stick to the inner wall of the cylinder 15, the proper operation of the piston 16 can easily release the non-return valve member 35. Where the fixing ring 27 is taken off and the piston 16 is pulled out, then the non-return valve member 35 can be drawn out at the same time. Further, said non-return valve member 35 can be easily removed from the piston 16. Therefore, the above-mentioned members can be easily washed, thereby keeping the non-return valve member 35 clean. When worn out beyond a certain extent, the non-return valve member 35 can be easily replaced by a fresh one. The inner wall of the cylinder 15 is used as the seat of the non-return valve member 35, it is unnecessary to provide an additional seat for said valve member 35, thereby simplifying the construction of the air supply and water supply changeover valve device 14.

Description is now given with reference to Fig. 3 of an air-supply and water supply changeover valve device according to a second embodiment of this invention. With this embodiment, the piston 16 and non-return valve member 35 are integrally prepared from elastic material. A flange 45 is integrally formed with the head 25 of the piston 16. The outer edge 46 of the flared flange 45 is pressed against the outer surface of the control section 2. The flared flange 45 elastically urges the piston 16 to a waiting position. In other words, the flared flange 45 has the same function as that of the compression coil spring 26 of the preceeding embodiment. Therefore, the parts of Fig. 3 the same as those of Fig. 2 are denoted by the same numerals, description thereof being omitted.

With the second embodiment of Fig. 3, all the movable parts are integrally formed, making it unnecessary to provide packing for assuring a waterproof and airtight construction. Therefore, the air-supply and water-supply changeover valve device has a simple construction and can be easily manufactured, offering further advantages that the surface of the piston 16 is freed of unnecessary narrow grooves or spaces, and is less likely to be soiled, and allow for easy washing.

Description is now given with reference to Fig. 4 of an air-supply and water-supply changeover valve device according to a third embodiment of this invention. The third embodiment of Fig. 4 is the same as the second embodiment of Fig. 3 in that all the movable parts related to the cylinder 15 are integrally formed. However, the third embodiment of Fig. 4 is different from the second embodiment of Fig. 3 in that the connection of the air ducts 8 and 12 on the cylinder 15 is secured with respect to each other and that the non-return valve member 35 has its flared portion 43 upturned, unlike the embodiment of Fig. 3, towards the open end of the cylinder 15. That is, the edge 43 of the flared portion 38 extends toward the open end side of the cylinder 15. The leak hole 33 extends straight through the central portion of the piston 16 axially of the piston 16. The air supply tube 12 communicates with the outside of the endoscope 1 through the bottom space 22 of the cylinder 15 and leak hole 33.

According to this embodiment, therefore, a new piston 16 can be easily inserted into the cylinder 15 during the replacement of pistons without involving the catching of the edge 43 of the non-return valve member 35 inside the cylinder 15. The construction of this embodiment can prevent injury to the non-return valve member 35 and omit the communication hole 31 as shown in the embodiment of Fig. 2, making it easier to manufacture the unit.

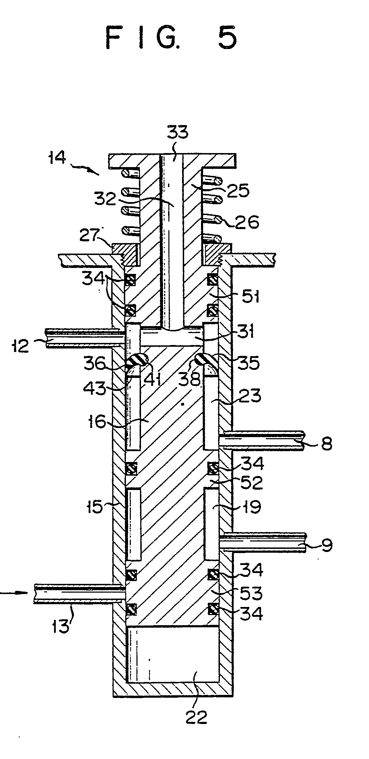

In an embodiment shown in Fig. 5, unlike the above-mentioned embodiment, air guide tube 8 and air supply tube 12 are located on the open end side of the cylinder 15 and water guide tube 9 and water supply tube 13 are positioned at the side of a bottom space 22 in the cylinder 15. An air conduit 23 for air supply is defined at the open end side of the cylinder 15, while a water supply conduit 19 for water supply is formed at the side of the bottom space 22 in the cylinder 15. First, second and third partitioning projected portions 51, 52, 53 are formed, in that order, on the outer surface of the piston 16 as viewed from the open end side of the cylinder 15. With the piston 16 in the ready state, the air supply tube 12 communicates with the air guide tube 8 through the air conduit 23. A non-return valve member 35 is fitted to the intermediate peripheral surface of the piston 16, such that it is located partway of the air conduit 23. The non-return valve member 35 has a flared portion 36 pressure-contacted with the inner surface of the cylinder 15 and a base portion 38. The non-return valve member 35 is adapted to normally shut off the air conduit 23. An outer edge 43 of the flared portion 36 extends at the side of a bottom space 22 in the cylinder 15. A recess 41 is formed in that peripheral surface portion of a piston 16 facing the air conduit 23, and the base portion 38 of the valve member 35 is elastically firmly fitted into the recess 41. With the piston 16 in the ready state, the open end of the air guide tube 8 is located near the stepped portion 52 such that it faces the air conduit 23. The open end of the air supply tube 12 is located at the open end side of the cylinder 15. The non-return valve member 35 is interposed between the open end of the air tubes 8, 12. The open end of the air supply tube 12 communicates with a leak hole 33 of the piston 16 without interposing the valve member 35. When the piston 16 is pushed into a cylinder 15, the open end of the air supply tube 12 is blocked by the outer surface of the stepped portion 51, while the open end of the air guide tube 8 remains opposite to the air conduit 23. Even when the piston 16 is pushed into the cylinder 15, the valve member 35 does not reach the open end of the air guide tube 8. With the piston 16 in the ready state, the open end of the water supply tube 13 is blocked by the outer surface of the third partitioning projected portion 53, while the open end of the water guide tube 9 is in communication with the water conduit 19. When the piston is pushed into the cylinder, the open ends of the water guide tube 9 and water supply tube 13 communicate with each other through water supply conduit 19.

Packings 34 are each placed in a water-tight fashion on the side surface of the projected portions 51, 52 and 53.

With the piston 16 in the ready state as shown in Fig. 5, air from the air supply tube 12 leaks into the outside of the endoscope 1 through the leak hole 33, thereby preventing the air from pushing aside the flared portion 38 and flowing toward the air guide tube 8. The open end of the water supply tube 13 is blocked by the outer surface of the third projected portion 53, preventing the supply of water. An air supply operation is performed by blocking the open end of the leak hole 33 by the finger of the operator. Since at this time the leakage of air through the leak hole 33 is prevented, the pressure of the air is increased, pushing aside the flared portion 36 of the valve member 35 to permit the air to flow into the air guide tube 8. The water supply operation is performed by pushing the piston 16 into the cylinder 15 with the open end of the leak hole 33 blocked by the finger of the operator. The open end of the air supply tube 12 is blocked by the outer surface of the projected portion 51, stopping the supply of the air. At the same time, the open ends of the water guide tube 9 and the water supply tube 13 communicate with each other through the water supply conduit 19, permitting water to flow from the water supply tube 13 into the water guide tube 9 through the water supply conduit 19. Because air in the bottom space 22 in the cylinder 15 is hermetically sealed by the packing 34 on the side surface of the third projected portion 53, if the piston 16 is further pushed into the cylinder 15, a reaction force is developed due to the compression of the air in the bottom space 22 in the cylinder 15. If at this time the finger of the operator is released away from the piston 16, the piston 16 is automatically returned to the original ready state owing to a resultant force of the reaction force and elastic force of a spring 26.

Where the pressure in the body cavity is increased owing to the supply of the air or the water, water, air or mucus tends to flow back into the air guide tube 8 at the side of the insertion section 3. However, such backflow is prevented as in the case of the above-mentioned embodiment, as the outer edge 43 of the flared portion 36 of the non-return valve member 35 is pressed against the inner wall of the cylinder 15.

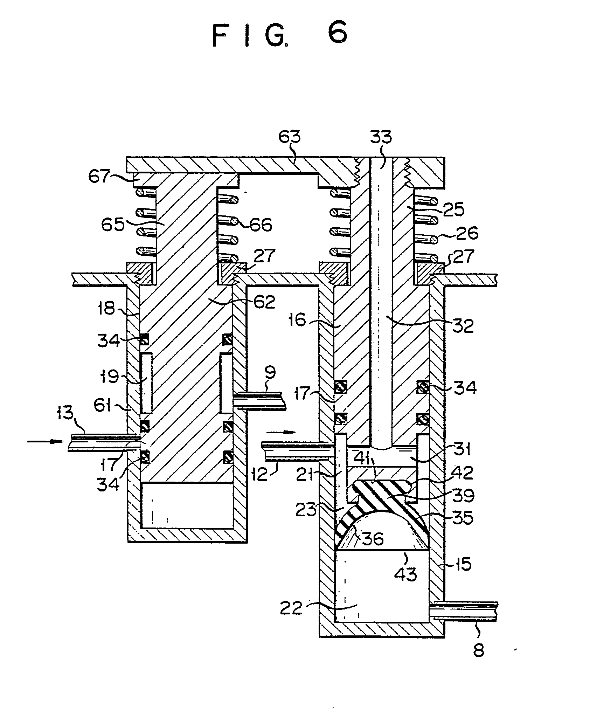

In an embodiment as shown in Fig. 6, in addition to a cylinder 15 and piston 16 for air supply, a cylinder 61 and piston 62 are provided for switching to the supply of water. An assembly of the cylinder 15 and piston 16 includes an arrangement necessary for the air supply operation which is similar to that in the embodiment as shown in Fig. 2. Likewise, an assembly of the cylinder 61 and piston 62 includes an arrangement necessary for the water supply operation which is similar to that in the embodiment as shown in Fig. 2. A detail of these arrangements are omitted with like numerals added to designate like parts or elements in Fig. 1. An operation member 63 is mounted on a head 25 of the piston 16. The portion of the operation member 63 extends onto the top of a head 65 of the piston 62. When the piston 16 is pushed into the cylinder 15, it is moved, together with the operation member 63, as a unit, causing the piston 62 to be depressed at the same time. A return spring 66 is wound around the outer peripheral portion of the head 65 of the piston 62 such that it is located between a fixing ring 27 and a flange 67. The return spring 66 serves to return the piston 62 to the original ready state. If an operation is effected as in the embodiment shown in Fig. 2, it is possible to perform a switching to air supply or water supply in a like manner.

As mentioned above, according to this invention the non-return valve member is always moved, together with the piston, as a unit in the cylinder. This firmly prevents the non-return valve member from being caught in the cylinder. Even if being caught there, it can be readily slipped along the inner wall of the cylinder. As the inner wall of the cylinder acts as a valve seat for the non-return valve member device, a simpler construction results and it is possible to remove the non-return valve member, together with the piston, as a unit away from the cylinder, permitting the ready washing of nearby parts including the piston.

**Claims**

1. An endoscope comprising:
a) an elongate insertion section (3) having distal and proximal ends (5, 10) and adapted to be inserted into a body cavity;
b) a control section (2) connected to the proximal end (10) of the insertion section (3);
c) fluid guide means comprised of an air guide tube section (8) and water guide tube section (9), having one end opened at the distal end (5) and the other end extending into the control section (2), and extending through the insertion section (3);

d) an air supply tube (12) provided to the control section (2) for flowing air;

e) a water supply tube (13) provided to the control section (2) for permitting the supply of water; and

f) air-supply and water-supply changeover means for permitting the air supply tube (12) to be connected to the air guide tube section (8) or the water supply tube (13) to be connected to the water guide section (9) of the fluid guide means, said air-supply and water-supply changeover means comprising:

(i) cylinder means (15) where one end of the air guide tube section (8) is disposed away from one end of the air supply tube (12) to permit a fluid communication to be made therebetween;

(ii) piston means (16) slidably inserted into the cylinder means (15) and forming in one part a water-supply conduit (19) and in another part an air-supply conduit (23) to permit switching from air supply to water supply to be effected between a ready position and operative position respectively;

(iii) means for urging the piston means (16) into the ready position;

(iv) being characterized in that a non-return valve member (35) is provided within the cylinder means (15) in the air supply conduit (23) such that it is located between said one end of the air supply tube (12) and said one end of the air guide tube section (8) of the fluid guide means, said non-return valve member (35) being provided on the outer surface of the piston means (16), and together with the piston means (16) being slidably movable, as a unit, against the walls of the cylinder means (15) according to the movement of the piston means (16), the valve member preventing the backflow of a fluid from the air guide section (8), while permitting a flow of air from the air supply tube (12) through the valve member (35).

2. An endoscope (1) according to claim 1, wherein the said device further includes a leak hole (33) having one end communicating with the air supply tube (12) without interposing the non-return valve member (35) and the other end communicating with the outside of the endoscope (1).

3. An endoscope (1) according to claim 2, wherein the leak hole (33) is arranged in the piston (16) and without interposing the non-return valve member (35).

4. An endoscope (1) according to claim 1, wherein said non-return valve member (35) has an engaging portion (39) and said piston means (16) has an engaging means (42) engaged with engaging portion (39) of the non-return valve member (35) to fix the latter to the former.

5. An endoscope (1) according to claim 1, wherein said non-return valve member (35) and said piston means (16) are integrally formed of an elastic material.

6. An endoscope (1) according to claim 1, wherein said non-return valve member (35) is located at the bottom end of the piston means (16).

7. An endoscope (1) according to claim 1, wherein said piston means (16) has a head portion (25) outwardly projecting out of the cylinder means (15) and having an elastic flange (45) formed of an elastic material and serving as urging means, said piston means (16) being urged under an elastic force of said urging means into the original ready position.

8. An endoscope (1) according to claim 7, wherein said piston means (16), said non-return valve member (35) and said flange (45) are integrally formed of an elastic material.

9. An endoscope (1) according to claim 1, wherein said air-supply and water-supply changeover means includes two separate mechanisms, one comprised of a cylinder (15) and piston (16) for switching to air supply and the other comprised of a cylinder (61) and piston (62) for switching to water supply.

**Patentansprüche**

1. Endoskop mit:

a) einem langgestreckten Einführabschnitt (3), der ein distales und ein proximales Ende (5, 10) aufweist und dafür vorgesehen ist, in eine Körperhöhlung eingeführt zu werden;

b) einem Steuerabschnitt (2), der mit dem proximalen Ende (10) des Einführabschnittes (3) verbunden ist;

c) Einrichtungen zum Leiten eines Fluides mit einem Luftleit-Rohrabschnitt (8) und einem Wasserleit-Rohrabschnitt (9), deren eines Ende sich zu dem distalen Ende (5) und das andere Ende in den Steuerabschnitt (2) öffnet und sich durch den Einführabschnitt (3) erstrecken;

d) einem Luftzufuhrrohr (12), das in dem Steuerabschnitt (2) angeordnet ist, um Luft zu leiten;

e) einem Wasserzufuhrrohr, das in dem Steuerabschnitt (2) angeordnet ist, um die Zufuhr von Wasser zu ermöglichen; und

f) Luftzufuhr- und Wasserzufuhr-Umschalteinrichtungen, die es erlauben, das Luftzufuhrrohr (12) mit dem Luftleit-Rohrabschnitt (8) zu verbinden, oder das Wasserzufuhrrohr (13) mit dem Wasserleit-Rohrabschnitt (9) der Fluidleit-Einrichtungen zu verbinden, wobei die Umschalteinrichtungen für Luftzufuhr und Wasserzufuhr folgendes aufweisen:

i) einen Zylinder (15), an dem ein Ende des Luftleit-Rohrabschnittes (8) von einem Ende des Luftzufuhrrohres (12) entfernt angeordnet ist, so daß eine Fluidverbindung zwischen diesen beiden Enden hergestellt werden kann;

ii) einen Kolben (16), der gleitbeweglich in dem Zylinder (15) eingesetzt ist und einerseits eine Wasserzufuhr-Leitung (19) und andererseits eine Luftzufuhr-Leitung (23) bildet, so daß ein Umschalten von Luftzufuhr auf Wasserzufuhr zwischen einer Startposition bzw. einer Arbeitsposition möglich ist; und

iii) eine Einrichtung, welche den Kolben (16) in die Startposition zwingt; dadurch gekennzeichnet

iv) daß ein Rückschlagventil (35) innerhalb des Zylinders (15) in der Luftzufuhr-Leitung (23) derart angeordnet ist, daß es zwischen dem einen Ende des Luftzufuhrrohres (12) und dem einen Ende des Luftleit-Rohrabschnittes (8) der Fluidleit-Einrichtung zu liegen kommt, wobei das Rückschlagventil (35) auf der äußeren Oberfläche des Kolbens (16) angeordnet ist und zusammen mit dem Kolben (16) als eine Einheit gegen die Wände des Zylinders entsprechend der Bewegung des Kolbens (16) gleitend beweglich ist und das Rückschlagventil verhindert, daß ein Fluid aus dem Luftleit-Rohrabschnitt (8) zurückfließt und es erlaubt, daß Luft von dem Luftzufuhrrohr (12) durch das Ventil (35) strömt.

2. Endoskop (1) nach Anspruch 1, dadurch gekennzeichnet, daß weiterhin ein Durchgangsloch (33) vorgesehen ist, dessen eines Ende mit dem Luftzufuhrrohr (12) ohne Dazwischenschalten des Rückschlagventils (35) in Verbindung steht und dessen anderes Ende mit der Außenseite des Endoskopes (1) in Verbindung steht.

3. Endoskop (1) nach Anspruch 2, dadurch gekennzeichnet, daß das Durchgangsloch (33) in dem Kolben (16) ohne Dazwischenschaltung des Rückschlagventils (35) angeordnet ist.

4. Endoskop (1) nach Anspruch 1, dadurch gekennzeichnet, daß das Rückschlagventil (35) einen Eingriffsabschnitt (39) aufweist und der Kolben (16) eine Eingriffsvorrichtung (42) aufweist, welche mit dem Eingriffsabschnitt (39) des Rückschlagventiles in Eingriff ist, um die beiden Teile miteinander zu verbinden.

5. Endoskop (1) nach Anspruch 1, dadurch gekennzeichnet, daß das Rückschlagventil (35) und der Kolben (16) einstückig aus einem elastischen Material gebildet sind.

6. Endoskop (1) nach Anspruch 1, dadurch gekennzeichnet, daß das Rückschlagventil (35) an der Unterseite des Kolbens (16) angeordnet ist.

7. Endoskop (1) nach Anspruch 1, dadurch gekennzeichnet, daß der Kolben (16) ein Kopfteil (25) aufweist, das aus dem Zylinder (15) nach außen vorspringt und einen elastischen Flansch (45) aufweist, der aus einem elastischen Material gebildet ist und als Vorspanneinrichtung dient, wobei der Kolben (16) aufgrund einer elastischen Kraft der Vorspanneinrichtung in die Startposition gezwungen wird.

8. Endoskop (1) nach Anspruch 7, dadurch gekennzeichnet, daß der Kolben (16), das Rückschlagventil (35) und der Flansch (45) einstückig aus einem elastischen Material gebildet sind.

9. Endoskop (1) nach Anspruch 1, dadurch gekennzeichnet, daß die Umschalteinrichtung für Luftzufuhr und Wasserzufuhr zwei getrennte Mechanismen aufweist, wobei einer der Mechanismen aus einem Zylinder (15) und einem Kolben (16) gebildet ist, um die Luftzufuhr zu schalten und der andere Mechanismus aus einem Zylinder (61) und einem Kolben (62) gebildet ist, um die Wasserzufuhr zu schalten.

**Revendications**

1. Endoscope constitué par:

a) une section d'insertion allongée (3) ayant des extrémités distale et proximale (5, 10) et pouvant être introduite à l'intérieur d'une cavité du corps humain;

b) une section de commande (2) reliée à l'extrémité proximale (10) de la section d'insertion (3);

c) des dispositifs de guidage de fluides constitués par une section (8) de tube de guidage d'air et une section (9) de tube de guidage d'eau, ayant une extrémité ouverte du côté de l'extrémité distale (5) et l'autre extrémité qui se prolonge dans la section de commande (2) et traverse la section d'insertion (3);

d) un tube d'arrivée d'air (12) installé dans la section de commande (2) pour faire circuler l'air;

e) un tube d'arrivée d'eau (13) installé dans la section de commande (2) pour faire circuler l'eau appliquée; et

f) un moyen de commutation de l'application d'air et de l'application d'eau permettant de relier le tube d'arrivée d'air (12) à la section du tube de guidage d'air (8) ou le tube d'arrivée d'eau (13) à la section de guidage d'eau (9) du dispositif de guidage de fluides,

ce moyen de commutation de l'application d'air et de l'application d'eau comprenant:

(i) un cylindre (15) sur lequel est branchée une extrémité de la section du tube de guidage d'air (8) en une position écartée de l'extrémité du tube d'application d'air (12) de façon à permettre la communication du fluide entre ces deux positions;

(ii) un piston (16) coulissant à l'intérieur du cylindre (15) et formant, d'une part, un conduit d'application d'eau (19) et, d'autre part, un conduit d'application d'air (23) qui assure la commutation de l'application d'air à l'application d'eau quand il passe de la position disponible à la position fonctionnelle respectivement;

(iii) un dispositif poussant le piston (16) en position disponible;

(iv) caractérisé en ce qu'un clapet anti-retour (35) est installé à l'intérieur du cylindre (15) dans le conduit d'arrivée d'air (23) de telle sorte qu'il se trouve entre l'extrémité du tube d'application d'air (12) et l'extrémité de la section de tube de guidage d'air (8) du dispositif de guidage de fluides, ce clapet anti-retour (35) étant disposé sur la surface extérieure du piston (16), et conjointement avec ce piston, pouvant coulisser, comme un ensemble d'une seule pièce, contre les parois du cylindre (15) selon le mouvement du piston (16), le clapet empêchant le refoulement d'un fluide venant de la section de guidage d'air (8) tout en permettant l'écoulement de l'air depuis le tube d'arrivée d'air (12) à travers le clapet (35).

2. Endoscope (1) selon la revendication 1, caractérisé en ce qu'il présente aussi un orifice de fuite (33) dont une extrémité communique avec le tube d'application d'air (12) sans interposition du clapet anti-retour (35) et dont l'autre extrémité communique avec l'extérieur de l'endoscope.

3. Endoscope (1) selon la revendication 2, caractérisé en ce que l'orifice de fuite (3) est ménagé dans le piston (16) et sans interposition du clapet anti-retour (35).

4. Endoscope (1) selon la revendication 1, caractérisé en ce que le clapet anti-retour (35) présente une portion d'accrochage (39) et en ce que le piston (16) présente un renfoncement (42) dans lequel s'introduit la portion d'accrochage (39) du clapet anti-retour (35) de façon à maintenir le clapet sur le piston.

5. Endoscope (1) selon la revendication 1, caractérisé en ce que le clapet anti-retour (35) et le piston (16) sont fabriqués d'une seule pièce en un matériau élastique.

6. Endoscope (1) selon la revendication 1, caractérisé en ce que le clapet anti-retour (35) est placé à l'extrémité inférieure du piston (16).

7. Endoscope (1) selon la revendication 1, caractérisé en ce que le piston (16) comprend une tête (25) dépassant à l'extérieur du cylindre (15) qui présente une collerette élastique (45) en un matériau élastique et sert de poussoir, ce piston (16) étant ramené sous l'effet de la force élastique de ce poussoir à sa position originale de disponibilité.

8. Endoscope (1) selon la revendication 7, caractérisé en ce que le piston (16), le clapet anti-retour (35) et la collerette (45) sont formés d'une seule pièce en un matériau élastique.

9. Endoscope (1) selon la revendication 1, caractérisé en ce que la vanne de commutation entre l'application d'air et l'application d'eau comprend deux mécanismes distincts, le premier constitué d'un cylindre (15) et d'un piston (16) qui commutent l'application d'air et le second constitué d'un cylindre (61) et d'un piston (62) qui commutent l'application d'eau.

# FIG. 1

# FIG. 2

FIG. 3

FIG. 4

2

# F I G. 5

F I G. 6

4